Europäisches Patentamt

(19) European Patent Office

Office européen des brevets

(11) EP 0 805 162 A1

(12) EUROPEAN PATENT APPLICATION

(43) Date of publication:
05.11.1997 Bulletin 1997/45

(51) Int. Cl.⁶: C07K 14/47, A61K 38/17

(21) Application number: 97106788.9

(22) Date of filing: 17.08.1988

(84) Designated Contracting States:
AT BE CH DE ES FR GB GR IT LI LU NL SE

(30) Priority: 17.08.1987 US 86694

(62) Document number(s) of the earlier application(s) in accordance with Art. 76 EPC:
88307608.5 / 0 304 279

(71) Applicant:
THE BOARD OF TRUSTEES OF
THE LELAND STANFORD JUNIOR UNIVERSITY
Stanford California 94305 (US)

(72) Inventors:
• Steinman, Lawrence
Palo Alto, California 94301 (US)
• Zamvil, Scott
Belmont, California 94002 (US)

(74) Representative:
Kiddle, Simon John et al
Mewburn Ellis,
York House,
23 Kingsway
London WC2B 6HP (GB)

Remarks:
This application was filed on 24 - 04 - 1997 as a divisional application to the application mentioned under INID code 62.

(54) **Peptide determinant associated with immunity**

(57) Novel peptides are provided involving sequences from proteins of interest which include a motif comprising a charged amino acid, followed by two hydrophobic amino acids, followed in the next or succeeding position by a polar amino acid, where the charged or polar amino acid may be substituted by glycine. Peptides having the motif are found to be stimulatory to T-cells and allow for modulation of immune response, as illustrated by tolerizing the host to neurological proteins.

EP 0 805 162 A1

## Description

Modulation of immune response is achieved employing peptides which include a "motif" in the sequence. The motif defines sequences of an immunogen which can be used for modulating the immune response to an antigen. Specific motifs are avoided in relation to endogenous host proteins.

Mammalian species have evolved in association with pathogens, where the host has had to evolve mechanisms to protect itself for survival. The immune system, which is the primary protective mechanism, has had to achieve the ability to respond to a wide variety of life threatening situations, involving not only pathogens, but also endogenous diseases such as tumors and genetic defects. In developing protective mechanisms, the immune system has developed a series of different pathways, where the individual pathways may respond to different stimuli to protect the host. Many of the pathways of the immune system are associated with detection of a foreign antigenic substance, which may then stimulate B-cells or T-cells to prevent invading pathogens from proliferating and to remove toxic substances.

Despite the extraordinary spectrum of antigens with which the immune system must contend, the immune system must be able to distinguish between self and nonself. In many instances, there is a failure of the immune system resulting in attacks made on endogenous proteins and/or cells. Such autoimmune diseases may be extremely debilitating and can lead to death.

The fact that pathogens are able to invade a mammalian host and cause disease is evidence of the fact that while in most cases the immune system is able to prevent the infection from being mortal, the immune system must cope with the infection after it has become established. Since in many cases, diseases can be not only debilitating, but have permanent effects, there is substantial interest in preventing the establishment of a pathogen, such as a virus, bacteria, or other microorganism. Toward this end, vaccines have been developed which activate the immune system, so that upon invasion by the targeted pathogen, the immune system is able to respond rapidly and protect the host. There is also interest in modulating the immune system to allow for organ transplants, inhibit autoimmune diseases, and enhance the host response to neoplasia or other native cell diseased state. Therefore, there is substantial interest in developing techniques and compositions which will allow for improved protection against a variety of diseases.

### Relevant Literature

Steinman, et al., Nature (1977) 265:173-175, describes the regulation of autosensitization to encephalitogenic myelin basic protein by macrophage-associated insoluble antigen.

Steinman, et al., Neurology (1980) 30:755-759, describes a study involving soluble protein in evaluating cell tolerance to myelin basic protein. Jahnke, et al., Science (1985) 229:282-284, reports a comparison of sequence homology between certain viral proteins and proteins related to encephalomyelitis and neuritis. Zamvil, et al., Nature (1985) 317:355-358, describe induction of chronic relapsing paralysis and demyelination by T-cell clones specific for myelin basic protein. Zamvil, et al., Nature (1986) 324:258-260, describe a T-cell epitope of the autoantigen myelin basic protein that induces encephalomyelitis. Sriram, et al., Cellular Immunology (1983) 75:378-382, describe the effect of intravenous administration of mouse myelin basic protein coupled to syngeneic spleen cells to prevent the induction of experimental allergic encephalitis (EAE). See also Sette et al. Nature (1987) 328:395 who report sequences coming within the subject motif.

### SUMMARY OF THE INVENTION

Short chain oligopeptides, by themselves or joined to other peptides, which oligopeptides include a sequence having a defined motif are employed for modulating the immune system. The motif comprises a charged amino acid, followed by two hydrophobic amino acids, followed in the fourth or fifth position by a polar amino acid, where the charged and polar amino acids may be substituted by glycine. Sequences of immunogens of interest are selected having this motif. Compositions are provided comprising one or more oligopeptides for modulating the immune system in vitro or in vivo. Those sequences analogous to motifs of endogenous proteins, particularly neuroproteins, are excluded from compositions to be administered to a host. The compositions find a broad spectrum of uses in modulating the immune system to affect the host response to antigens.

### DESCRIPTION OF THE SPECIFIC EMBODIMENTS

Novel oligopeptides and their use are described for modulating the immune system in relation to a particular immunogen. The oligopeptides comprise an amino acid sequence defined by a "motif" present in the immunogen of interest. The motif-containing oligopeptide will have at least about 9 amino acids and need not have more than about 15 amino acids of the sequence of the immunogen, usually having the defined motif sequence as other than the C-terminal sequence of the oligopeptide sequence. One or more sequences may be identified in the same immunogen having the defined motif sequence. Oligopeptides containing these sequences may be used in a single composition to be effective with a plurality of haplotypes. Sequences having substantial homology with endogenous proteins, particularly neuroproteins, are excluded. The immunogen motif sequence containing fragment may be used by itself, as part of a larger fragment, where the immunogen is joined to other than the wild type sequence, or may be joined covalently to other organic molecules, either proteinaceous or non-proteinaceous.

In referring to an epitope, the epitope will be the basic element or smallest unit of recognition by a receptor, particularly immunoglobulins and T-cell receptors, where the sequence may be contiguous or non-contiguous, and the amino acids essential to the receptor recognition may be contiguous and/or non-contiguous in the sequence. T-cell epitopes involve contiguous residues, while immunoglobulin epitopes are conformational determinants, and may be either contiguous or non-contiguous.

The immune response which is modulated is predicated upon a ternary complex, involving a cell having a transplantation antigen, a T-cell receptor restricted by the transplantation antigen, and a polypeptide which specifically binds to the combination of transplantation antigen and T-cell receptor. The transplantation antigens may be divided into two classes, Class I and Class II. Class I is relatively ubiquitous, found on most nucleated cells of a mammalian host, and has a polymorphic 45 Kd transmembrane protein which is non-covalently associated with a 12Kd non-polymorphic protein "$\beta_2$ microglobulin." By contrast, Class II transplantation antigens are restricted to relatively few sets of cells, primarily lymphocytes, macrophages, and dendritic cells, and comprise polymorphic $\alpha$- and $\beta$-chains. In the case of Class I transplantation antigens, one is primarily concerned with cellular aberrations or diseased states, such as viral infection, mycoplasma infection, neoplasia, or the like, or with organ transplants. The T-cells involved will normally cause the destruction of the aberrant cell.

By contrast, Class II transplantation antigens are concerned with activation of the cellular immune system, resulting in the expansion of cells involved with protection of the host against aberrant physiological states. The aberrant physiological states may be involved with pathogenic invasions, including viruses, bacteria, fungii, protista, toxins, or the like. In addition, the Class II cells may be involved with various autoimmune diseases, such as rheumatoid arthritis, systemic lupus erythematosus, diabetes, multiple sclerosis, etc., as well as those cellular aberrations or diseased states associated with Class I transplantation antigens.

Class I transplantation antigens are also involved in rejection of organ transplants. The immune system is able to recognize the foreign nature of the transplantation antigens present on the transplanted organ and attack the organ. In this situation, one does not wish to protect the host from the foreign cells, but rather diminish the subset of the immune system which is specific for attacking the organ transplant.

In many situations, there will also be interest in controlling lymphocyte response in vitro. These situations may involve specifically destroying cells infected with mycoplasma or virus, removing a particular subset of cells from a mixture of neoplastic and normal cells, expanding a particular subset or subsets of cells in a mixture, providing for conditioned medium for production of various lymphokines, such as IL-2, or the like. The in vitro system may involve whole blood, plasma, serum, cellular fractions, normal or immortalized cells, etc.

The compositions of this invention will comprise oligopeptides, one or more different oligopeptides, comprising the following sequence: charged amino acid, two hydrophobic amino acids, and at least one of the next two amino acids being a polar amino acid, where the charged or polar amino acid may be substituted by glycine, usually not more than one being substituted by glycine. The charged amino acids are aspartic acid, glutamic acid, lysine, arginine, and histidine (D, E, K, R, H). The hydrophobic amino acids are alanine, proline, valine, leucine, isoleucine, methionine, phenylalanine, tryptophan, and tyrosine, that is both the aliphatic and aromatic neutral or substantially neutral amino acids having not more than one heteroatom, e.g., chalcogen, on the side chain (A, P, V, L, I, M, F, W, and Y). The polar amino acids will be the charged amino acids, as well as serine, threonine, asparagine, and glutamine (S, T, N, and Q).

The motif sequence will be part of a sequence of an immunogen of interest, there usually being more than one partial sequence in the immunogen including the subject motif. The oligopeptide comprising the subject motif may be from any site of the immunogen sequence, that is N-terminal or C-terminal proximal or central, where the oligopeptide sequence will normally be substantially homologous with from 9 to 15 amino acids of the immunogen sequence, although longer sequences may also be employed. Usually the difference in homology will not be more than 2 non-conservative lesions, more usually not more than 2 lesions, which may be insertions, deletions, conservative or non-conservative substitutions.

Usually, the motif sequence present in the oligopeptide will be at other than the C-terminus of the oligopeptide, desirably being at the N-terminus and not closer to the C-terminus than the center of the sequence, where the second, third, or fourth amino acid of the motif (depending upon whether there are four or five amino acids in the motif) is the central amino acid.

The compositions of this invention will include usually at least one sequence of an immunogen of interest including the subject motif and may include two or more oligopeptide motif containing sequences of from about 9 to 15 amino acids present in the immunogen, depending upon the number of motifs present in the immunogen. Thus, if there are a plurality of motifs present in the immunogen, all or fewer than all of the sequences including the motifs may be employed in a single composition. Usually, there will be not more than 10 different motif comprising oligopeptides, more usually not more than about 6 different oligopeptides in the composition.

The oligopeptides employed in this invention which define motif containing regions and are not joined to heterologous sequences (that is sequences other than the natural sequence to which they are normally joined), will not include cytochrome c, ovalbumin, myoglobin, nuclease from <u>Staphylococcal aureus</u>, lysozyme, repetitive sequences of 1, 2, and 3 amino acids, influenza hemagglutinin, naturally occurring hormones of fewer than about 20 amino acids, such as oxytocin, bradykinin and angiotensin, herpes glycoprotein d, insulin, particularly bovine insulin B-chain, rat myelin basic protein, ragweed allergen Ra3, human ACh receptor-$\gamma$, VP1 foot-mouth virus, angiotensin 2, fibrinopeptide B-14, minimum stimulatory polymer, HLA CW3, myelin basic protein, particularly rat and guinea pig, rabies glycoprotein, flu matrix protein, tuberculosis 65 kd protein, or other oligopeptide prepared prior to the effective filing date of the subject application.

In preparing the subject compositions, one would select an immunogen of interest against which an immune response of a host is to be modulated. Thus, the immunogen of interest may be a protein associated with a pathogen. Pathogens include viruses, microorganisms, such as bacteria, fungi, protista, etc. The immunogen of interest may be a capsid protein, an enzyme protein, an envelope protein, a surface membrane protein, or any other protein which may naturally induce an immune response, which aids in the protection of the host from the pathogen. Other proteins of interest will include mammalian proteins, such as transplantation antigens, surface membrane proteins associated with blood type, or other cellular protein which may be recognized by a host immune system as foreign. A third group of oligopeptides will be associated with proteins endogenous to the host, associated with autoimmune diseases, where the oligopeptide may serve to tolerize the host to prevent immune attack against the endogenous protein or cell producing the endogenous protein. Other immunogens will also be of interest.

The particular protein of interest will be screened for the presence of the subject motif and one or more sequences including the motif selected. Where the haplotype of the intended recipient is known, one sequence may be preferred over another. However, where the haplotype is not known, or the composition may be administered to a number of different hosts, it will frequently be desirable to combine a number of the sequences as oligopeptides in the same composition. The oligopeptides may be present as the individual peptides, or may be joined together in a single sequence, with or without intervening bridges, where any bridges will be other than the naturally occurring intervening sequences of the immunogen. Desirably, any such sequence would have fewer than about 100 amino acids, more usually fewer than about 60 amino acids.

Sequences which are excluded will be sequences from proteins endogenous to the host, which include such proteins as the neurological proteins found in the peripheral nervous system (PNS) or the central nervous system (CNS) and the acetylcholine receptor (AChR). These proteins are designated as $P_o$ which is found in the PNS and CNS, P1, in myelin basic protein, the pre-dominant CNS protein of myelin, P2, a predominant PNS myelin protein, PLP, a proteolipid protein, a PNS and CNS myelin constituent, and the acetylcholine receptor. P1 is involved in post-immunization encephalomyelitis and may be involved in multiple sclerosis. P2 is involved in post-immunization neuritis (Guillain-Barre syndrome) a major complication, for example, in the swine flu immunization program and the acetylcholine receptor is involved in myasthenia gravis and may play a role in post-immunization myositis.

In addition to oligopeptides including the subject motif, other oligopeptides which may be employed are those N-terminal peptides which are acetylated, where the only requirement is that the parent immunogen is present as the N-acetylated protein. Of particular interest for this purpose is the N-terminus of myelin basic protein, which has the sequence Ac-A-S-Q-K-R-P-S-Q-R-H-G-S-K-Y-L, other acetylated N-termini include the P2 protein, which has the sequence Ac-S-N-K-F-L-G-T-W-K-L-V-S-S-G.

The subject oligopeptides may be modified in a variety of ways. For tolerization, the subject peptides may be conjugated to syngeneic spleen cells, or be linked to an innocuous immunogen to which the host has been previously immunized, such as tetanus toxoid, bovine serum albumin, etc. Adjuvants are normally avoided. Alternatively, the oligopeptide may be used for immunization (Steinman <u>et al</u>., <u>Nature</u> (1977) <u>265</u>: 173-177; and Steinman <u>et al</u>., <u>Neurology</u> (1980) <u>30</u>:755-759). To enhance the immune response against a plurality of immunogens, which may be associated with the same or different organism, as already indicated, the subject oligopeptides may be joined to oligopeptides sharing the same or different motif of the same immunogen or associated with an epitope of a different immunogen. The epitope may or may not include the subject motif. Thus, the resulting polypeptide will be able to activate, inactivate or tolerize the immune system to a plurality of immunogens, which are associated with the same or different organism. This approach may have particular advantages where vaccines are employed against a battery of diseases, such as the TORCH complex, various children's diseases, diseases endemic to particular locations, and the like.

The subject oligopeptides may not only be bonded to other oligopeptides, or proteins, but may be conjugated to a variety of non-proteinaceous products, particularly lipopolysaccharides, polysaccharides, lipids, glycerides, and the like. In many instances, the compounds may be inactivated toxins, provide for production of neutralizing antibodies or activate various cytotoxic T-cells toward a particular pathogen.

Transplantation antigens have polymorphic regions, where the individual alleles are associated with specific hosts. For the most part, the host will be diploid and heterozygous, so that each host will have two haplotypes, meaning that there will be two different copies of a particular transplantation antigen type from the same locus, unless the host is homozygous at that particular locus.

Therefore, as to an individual host or a plurality of hosts, mixtures of oligopeptides will usually be employed.

The subject compositions find use in a variety of ways associated with the immune system. The subject compositions can be used as vaccines for activating the immune response to a particular pathogen or toxin. The subject compositions may be used to tolerize an individual, in the case of organ transplants, so that the rejection of the organ transplant or graft-v-host disease may be substantially reduced. The subject compositions may also be used to protect against autoimmune diseases, so that the immune system will be inhibited from attacking native proteins or host cells. The subject compositions may be used in the treatment of cancer. Other situations involving the immune system may also use the subject compositions with advantage.

Depending upon the particular application, the subject compositions may be administered in a variety of ways, by themselves or in conjunction with various additives. The subject compositions may be combined with various adjuvants, such as alum, BCG, oils, muramyl dipeptide. Alternatively, the subject compositions may be covalently bonded by any convenient linking group to an appropriate immunogen, such as tetanus toxoid, bovine gamma-globulin, keyhole limpet hemocyanin, etc. Various carriers may be employed which are physiologically acceptable, such as water, alcohol, saline, phosphate buffered saline, sugar, mineral oil, etc. Other additives may also be included, such as stabilizers, detergents, flavoring agents, thickeners, etc. The amount of active ingredient administered will vary widely depending upon the particular composition, the particular host, the number and frequency of administrations, the manner of administration, etc. Usually, there will be from about 0.01 to 10 $\mu$g/kg of host more usually from about 0.05 to 5 $\mu$g/kg of host, where the concentration may range from 10 $\mu$g/ml to 1 mg/ml.

The manner of administration may be varied widely, depending upon the formulation and nature of the active ingredient. Administration may be parenteral, intravascular, peritoneally, subcutaneous, oral, etc, may employ catheters, pumps, constant diffusion membranes, etc.

The subject compositions may also be used for diagnostic purposes for detecting T-cells which bind to the oligopeptide. Thus, the subject oligopeptide may be used in conjunction with a transplantation antigen and T-cells to determine the size of the T-cell population stimulated by a particular sequence. The subject peptide may also be used in competitive determinations to compare binding affinity of oligopeptides.

The subject compositions may be prepared in a variety of ways. The oligopeptides may be synthesized in accordance with conventional synthetic techniques, particularly automated synthesizers, or may be prepared by recombinant techniques, where an appropriate DNA sequence is devised for insertion into an expression cassette for expression in a microorganism host. Particularly, where the oligopeptide will be joined to one or more other oligopeptides, the recombinant techniques may be employed with advantage.

A large number of expression vectors are commercially available or have been described in the literature, where the expression vectors include transcriptional and translational initiation and termination regions separated by a polylinker having a plurality of unique restrictions sites. Sequences may be prepared coding for the oligopeptide or polypeptide of interest, which sequence may be inserted into the polylinker for expression in the host. If desired, the sequence may be prepared with a signal sequence, so that the product is secreted and processed to provide for the mature polypeptide.

Alternatively, the polypeptide may be covalently conjugated to another polypeptide or other molecule by providing for an appropriate functionality which can be used as a site for linking. For example, a cysteine may be used to react with an activated olefin, e.g., maleimide, so as to form a thioether. Alternatively, the oligopeptide may be modified at it's N-terminus to provide for a linking site, particularly where the oligopeptide is synthesized, which may include a variety of functional groups. If convenient, the synthesized oligopeptide may be conjugated while still bound to a support, followed by removing the conjugate from the support. Alternatively, a tyrosine may be used, which may be linked through a diazo group. Other conventional techniques may also be employed.

The subject oligopeptides may be derived from a wide variety of organisms including pathogens, such as retroviruses, such as the human retroviruses HTLV1-4, Pseudomonas, _Bordatella pertussis_, herpes simplex virus I and II. A number of pathogens are listed in U.S. Patent No. 4,208,479, which disclosure is incorporated herein by reference.

The following sequences will normally be excluded as motifs for immunization, but may be used with advantage for tolerization by themselves or in conjunction with each other or other epitope sequences. G-A-P-S; G-A-V-G; E-W-V-S; K-V-P-T; G-V-V-L-G; G-A-V-I-G; G-I-L-G; K-A-A-S (associated with $P_0$); Ac-A-S-Q-K-R; K-Y-L-A-T; G-I-L-D; R-F-F-G; H-F-F-K; K-I-F-K (associated with $P_1$); Ac-S-N-K-F-L; K-F-L-G; K-L-V-S; E-Y-M-K; G-L-A-T; R-V-I-I-S; K-M-V-V-E; R-I-Y-E (associated with $P_2$); G-L-L-E; K-L-I-E; H-A-F-Q; G-A-V-R; K-W-L-G; K-F-V-G; R-M-Y-G; K-L-M-G (associated with PLP); and L-V-A-K; K-I-W-R; E-W-V-I-K; K-V-F-I-D; K-I-F-T; K-Y-I-A-E (associated with AChR). These sequences will normally be part of oligopeptide sequences of about 9 to 15 amino acids as described for the other motifs.

The following examples are offered by way of illustration and not by way of limitation.

EXPERIMENTAL

The determinant of myelin basic protein (MBP) P5-17 (Zamvil, _et al_., Nature, _supra_) contains a pattern P-S-Q-R-H-G-S-K-Y-L-A-T-A. Using the algorithm for the subject motif for predicting T-cell clones the epitope of the clone F1-28, a MBP-specific T-cell was isolated (Zamvil, et al., J. Exp. Med. (1985) 162: 2107), which clone recognizes the autoantigen myelin basic protein. A peptide corresponding to amino acids P35-47 containing two patterns GILD and RFFS was synthesized and shown to be stimulatory. The peptide determinant tested was G-I-L-D-S-I-G-R-F-F-S-G-D-R-G-A-P. The stimulatory epitope was shown with overlapping peptides to actually consist of L-D-S-I-G-R-F-F-S-G-D-R-G-A-P (Zamvil et al., Nature (1986) 324:258).

In another disease, myasthenia gravis, a T-cell epitope was discovered by using the subject algorithm to construct synthetic peptides of the acetylcholine receptor. The peptide AChR P215-232, D-T-P-Y-L-D-I-T-Y-H-F-V-M-Q-R-L-P-L was particularly stimulatory in a number of myasthenics. Other stimulatory peptides included 277-291 and 330-347 which followed the subject algorithm. Antigen-specific T-cell clones are isolated from peripheral blood lymphocytes (PBL), cultured in vitro with antigen and syngeneic irradiated PBL as antigen presenting cells (APL) (Cunningham et al., J. Gen. Virol. (1985) 66:249); Eckles et al., Nature (1981) 301:716).

To render autoimmunogenic peptides tolerigenic, these peptides may be conjugated to lymphocytes (Sriram, et al., 1983, supra) or by coupling the peptide to a carrier such as tetanus toxoid or bovine serum albumin, employing conventional linking groups (Herzenberg, et al., Ann. Rev. Imm. (1983) 1:609-632).

Synthetic MBP Peptides: Peptides corresponding to the amino acid sequences of rat (R) and bovine (B) MBP (Martenson, 1984, In Experimental Allergic Ensephalomyelitis. A Useful model for multiple sclerosis. Alvard, ed. Alan Liss, N.Y.), were synthesized as described previously using solid phase techniques (Erickson and Merrifield, 1976, In The Proteins Vol. 2, Neurath, ed. Academic Press, NY, p 255). Peptides were separated from the various organic side products and the purity was determined by high pressure liquid phase column (Merck, Darmstadt, Germany) and by amino acid analysis. These peptides were not further purified since they all contained greater than 90% of the desired product.

The subject peptides described above were employed in the following test procedure:

Proliferation Assay: Proliferative responses were determined as described previously (Zamvil et al., Nature (1985) 317:355). $1 \times 10^4$ T-cells were cultured with $5 \times 10^5$ X-irradiated (3,000 rad) PL/J splenic APC in 0.2 ml of culture media in 96 well flat-bottomed microtiter plates (Falcon, 3072). Peptides were added to culture giving the final concentrations indicated. At 48 hours incubation, each well was pulsed with 1 $\mu$Ci $^3$H-thymidine and harvested 16 hours later. The mean c.p.m. thymidine incorporation was calulated for triplicate cultures. Standard deviations from replicate cultures were within 10% mean value.

P35-47 of the human myelin basic protein (MBP) comprising the RFFS motif was found to be stimulatory with mouse T-cells restricted by MHC I-$E_\alpha^u E_\beta^u$; P5-17 of the human myelin basic protein comprising the motif KYLAT was found to be stimulatory with mouse T-cells restricted by I-$A_\alpha^s A_\beta^u$ or I-$A_\alpha^u A_\beta^u$; and P89-101 of the human myelin basic protein comprising the motif HFFK was found to be stimulatory with mouse T-cells restricted by I-$A_\alpha^s A_\beta^s$.

The algorithm can be used to define what immunogenic part of an autoantigen shares sequence homology with pathogens. For example, in the case of MBP P35-47 shared with paramyxoviruses and influenza and MBP 89-101 shared with picornavirus, the critical sequence triggering the T-cell is shared with a pathogen.

It is evident from the above results, that sequences may be identified and employed as peptides for modulating the immune system in a variety of ways. As one illustration, a host may be tolerized against autoimmunogenic peptides by employing the subject peptides as tolerogens in a tolerizing manner. In other cases one may wish to immunize the host, so as to inhibit any further attack against endogenous cells or components. The method further provides for protection against various diseases resulting from vaccination, by eliminating any sequences which relate to stimulatory sequences found in endogenous proteins, particularly proteins involving the nervous system.

All publications and patent applications mentioned in this specification are indicative of the level of skill of those skilled in the art to which this invention pertains. All publications and patent applications are herein incorporated by reference to the same extent as if each individual publication or patent application was specifically and individually indicated to be incorporated by reference.

Although the foregoing invention has been described in some detail by way of illustration and example for purposes of clarity of understanding, it will be obvious that certain changes and modifications may be practiced.

**Claims**

1. A polypeptide which comprises a human myelin basic protein peptide (hMBP) fragment including P89-101 of hMBP, excluding native hMBP protein.

2. The polypeptide of claim 1 wherein the polypeptide is fewer than 60 amino acids in length.

3. The polypeptide of claim 1 or claim 2 wherein the hMBP fragment is fewer than 15 amino acids in length.

4. The polypeptide of any one of claims 1 to 3 wherein the hMBP peptide fragment is modified by not more than two non-conservative lesions.

5. The polypeptide of any one of claims 1 to 4 wherein the hMBP peptide fragment is joined by chemical means to another epitope of hMBP.

6. The polypeptide of any one of claims 1 to 5 wherein the polypeptide is conjugated to an immunogen.

7. The polypeptide of any one of claims 1 to 6 for use in a method of medical treatment.

8. The polypeptide of claim 7 for use in tolerizing a mammalian host immune system comprising B cells and T cells to an immunogen of interest, wherein said immunogen is restricted by a transplantation antigen of said host.

9. The use of a polypeptide of any one of claims 1 to 6 in the preparation of a medicament for tolerizing a mammalian host immune system comprising B cells and T cells to an immunogen of interest, wherein said immunogen is restricted by a transplantation antigen of said host.

European Patent
Office

**EUROPEAN SEARCH REPORT**

Application Number

EP 97 10 6788

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | JOURNAL OF NEUROCHEMISTRY, vol. 46, no. 1, 1 January 1986, pages 47-53, XP000567823 CHOU CH -H J ET AL: "MONOCLONAL ANTIBODIES TO HYMAN MYELIN BASIC PROTEIN" * page 50, left-hand column, paragraph 2 - page 51, left-hand column, paragraph 1 * --- | 1,2,4,7 | C07K14/47 A61K38/17 |
| X | JOURNAL OF IMMUNOLOGY, vol. 130, no. 1, January 1983, BALTIMORE US, pages 191-194, XP002038675 R.B. FRITZ ET AL.: "Induction of experimental allergic encephalomyeltits in PL/J and (SJL/J x PL/J)F1 mice by myelin basic protein and its peptides: Localization of a second encephalitogenic determinant" * page 193, left-hand column, paragraph 2 - page 194, left-hand column, paragraph 1 * --- | 1,7 | |
| X | JOURNAL OF IMMUNOLOGY, vol. 130, no. 5, May 1983, BALTIMORE US, pages 2183-2186, XP002038676 C.-H. J. CHOU ET AL.: "Encephalitogenic activity of the small form of mouse myelin basic protein in the SJL/J mouse" * page 2186, left-hand column, paragraph 2 * --- | 1,2,4,7 | TECHNICAL FIELDS SEARCHED (Int.Cl.6) C07K A61K |
| T | WO 88 10120 A (BRIGHAM & WOMENS HOSPITAL) 29 December 1988 * claims; examples; table V * ----- | 1,7 | |

The present search report has been drawn up for all claims

| Place of search | Date of completion of the search | Examiner |
|---|---|---|
| THE HAGUE | 25 August 1997 | Fuhr, C |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

.............................................................................

& : member of the same patent family, corresponding
    document

EPO FORM 1503 03.82 (P04C01)